# EUROPEAN PATENT APPLICATION

(11) **EP 2 752 207 A1**
(43) Date of publication of application: **09.07.2014**
(21) Application number: 11871781.8
(22) Date of filing: 31.12.2011
(51) Int. Cl.: A61M 1/00, A61M 27/00, G01F 23/28

(54) **NEGATIVE PRESSURE WOUND THERAPY SYSTEM WITH ALARM FUNCTION FOR FLOW RATE OF EXUDATE**

(30) Priority: 29.08.2011 CN 201120318007 U
(71) Applicant: Foryou Multimedia Electronics Co. Ltd., Huizhou, Guangdong 516000 (CN)
(72) Inventor: JIAN, Jiqi, Huizhou Guangdong 516000 (CN)
(74) Representative: Maiwald Patentanwalts GmbH
(86) International application number: PCT/CN2011/085190
(87) International publication number: WO 2013/029330

(57) **Abstract**

A negative pressure wound therapy system with an alarm function for the flow rate of exudate comprises a liquid collecting bottle (5) for collecting wound exudate, a negative pressure wound therapy device (8) consisting of a negative pressure source (6) and a controller (7), and a wound kit for filling and enclosing the wound, which communicates with the liquid collecting bottle (5) via a connecting tube (4). The system further comprises a flow rate alarm processing module and an alarm warning device. If the flow rate of exudate is greater than a predefined value, the flow rate alarm processing module issues an alarm instruction. The alarm warning device sends a warning message according to the alarm instruction. If the flow rate of exudate is greater than the predefined value, the system issues an alarm instruction to the alarm warning device, by which a warning message is sent to remind medical staffs of timely inspection, thereby avoiding occurrences of serious accidents, such as the blood in human body being sucked out, and so on.

## Description

The present application claims the priority to Chinese Patent Application No. 201120318007.X, entitled "NEGATIVE PRESSURE WOUND THERAPY SYSTEM WITH ALARM FUNCTION FOR FLOW RATE OF EXUDATE", filed on August 29, 2011 with the Chinese State Intellectual Property Office, which is incorporated herein by reference in its entirety.

### FIELD

The present disclosure relates to the technical field of negative pressure wound therapy devices, and in particular to a negative pressure wound therapy system with an alarm for exudates flow velocity.

### BACKGROUND

The principle of negative pressure wound therapy devices (NPWT) is to promote wound healing by applying a negative pressure on a wound area. The clinical practice of the United States in the last decade shows that the device is very effective in healing acute and chronic wounds.

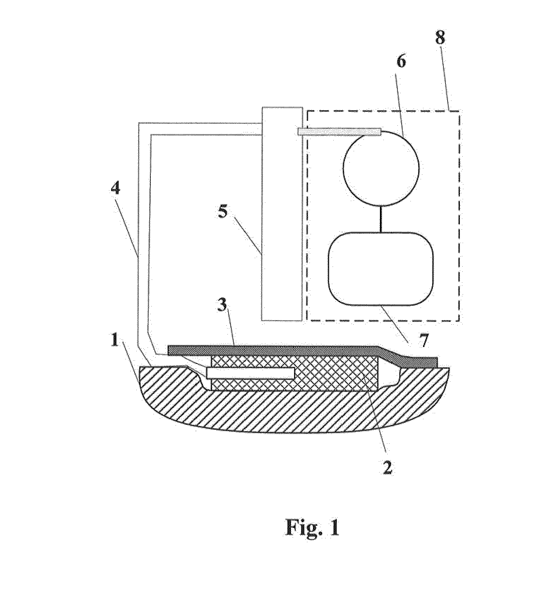

Generally, a negative pressure wound therapy system includes a negative pressure wound therapy device, a wound kit and a container for collecting wound exudates (refers to a fluid collection canister). The wound kit is adapted to enclose a wound and create a negative pressure space. The negative pressure wound therapy device, including a negative pressure source and a controller, is adapted to create a desirable negative pressure space for the wound. Figure 1 shows a common negative pressure wound therapy device, where a negative pressure wound therapy device 8 is schematically shown within the dashed box; the skin tissue around the wound is indicated by the reference number 1; the wound area is covered by a wound filler 2; a wound enclosure 3 is set above the wound filler 2 to enclose the wound, where the wound filler 2 is in communication with a fluid collection canister 5 via a catheter 4, hence the exudates from the wound may be guided into the fluid collection canister 5 which is adapted to collect the exudates from the wound. The other end of the fluid collection canister 5 is connected to a negative pressure source 6 which may provide a negative pressure for powering the flowing of the wound exudates into the fluid collection canister 5; the negative pressure source 6 is connected to a controller 7 which may control the negative pressure source 6.

The wound exudates, formed due to multiple physiological causes, are the humors accumulated around a wound area. In terms of the wound treatment, the wound exudates management is among the important subjects of the modem wound care research.

According to the research on the wound exudates in the modern wound care, the monitoring of wound exudates flow velocity is very important. Massive hemorrhage on a wound is frequent in the NPWT accidents, that is because the liquid flow is accelerated due to the negative pressure suction, and then the blood may outflow due to the negative pressure; therefore, the exudates flow velocity in the negative wound therapy system is required to be monitored, hence the above accidents may be prevented from happening. However, in the prior art, still by the medical staff, the volume of the exudates is checked and the change of the volume over time, i.e., the flow velocity, is recorded; if the medical staff fails to monitor the above parameters in time, the above accidents may happen.

### SUMMARY

It is to provide a negative pressure wound therapy system with an alarm for exudates flow velocity, which may monitor the wound exudates flow velocity and send out an alarm in abnormal conditions to facilitate the observation and research of medical staff and prevent accidents from happening.

In order to solve the above technical issues, the technical schemes of the disclosure are specified in the following.

A negative pressure wound therapy system with an alarm for exudates flow velocity, including a fluid collection canister adapted to collect wound exudates; a negative pressure wound therapy device including a negative pressure source and a controller; a wound kit adapted to fill and enclose the wound area, where the wound kit is in communication with the fluid collection canister via a catheter, where the system further includes a flow velocity alarm processing module and an alarming apparatus, where the flow velocity alarm processing module is adapted to send out an alarm instruction in a case that the exudates flow velocity exceeds a preset value; and the alarming apparatus is adapted to send out a warning signal according to the alarm instruction.

Preferably, the flow velocity alarm processing module includes: a monitoring unit adapted to monitor a volume of the exudates in the fluid collection canister and the duration in which the exudates flow into the fluid collection canister; an calculating unit adapted to figure out a current exudates flow velocity according to the change of the volume of the exudates within a set time period; and a processing unit adapted to send out an alarm instruction in a case that the current flow velocity exceeds a preset value.

Preferably, the preset value includes a first preset value and a second preset value, where the flow alarm processing module may send out a first alarm instruction if the exudates flow velocity exceeds the first preset value; the alarming apparatus sends out a warning signal according to the first alarm instruction; the flow alarm processing module may send out a second alarm instruction if the exudates flow velocity exceeds the second preset value; the alarming apparatus sends out a warning signal according to the second alarm instruction.

Preferably, the alarming apparatus includes an LED adapted to emit lights in a corresponding color after receiving the alarm instruction.

Preferably, the alarming apparatus includes a buzzer adapted to send out a sound at a preset frequency after receiving the alarm instruction.

Preferably, the alarming apparatus includes an LCD display interface adapted to display alarming information after receiving the alarm instruction.

Preferably, the alarming apparatus includes:
an LED, adapted to emit lights in a corresponding color after receiving the alarm instruction; and
a buzzer, adapted to send out a sound at a preset frequency after receiving the alarm instruction.

Preferably, the alarming apparatus includes:
an LED, adapted to emit lights in a corresponding color after receiving the alarm instruction; and
an LCD display interface, adapted to display alarming information after receiving the alarm instruction.

Preferably, the alarming apparatus includes:
a buzzer, adapted to send out a sound at a preset frequency after receiving the alarm instruction; and
an LCD display interface, adapted to display alarming information after receiving the alarm instruction.

Preferably, the alarming apparatus includes:
an LED, adapted to emit lights in a corresponding color after receiving the alarm instruction;
an LCD display interface, adapted to display alarming information after receiving the alarm instruction; and;
a buzzer, adapted to send out a sound in a preset frequency after receiving the alarm instruction.

Compared with the prior art, as the negative pressure wound therapy system with an alarm for exudates flow velocity is provided with an exudates flow velocity alarm processing module, the system may send out an alarm instruction to an alarming apparatus if the exudates flow velocity exceeds a preset value, and then the alarming apparatus may send out a warning signal to remind the medical staff, hence the fatal accident such as that the blood is sucked out may be prevented from happening.

### BRIEF DESCRIPTION OF THE DRAWINGS

Figure 1 is a schematic structure diagram of an existing negative pressure wound therapy system;
Figure 2 is a schematic structure diagram of a negative pressure wound therapy system with an alarm for exudates flow velocity provided according to the embodiment I of the disclosure;
Figure 3 is a schematic diagram of the cooperation among an exudates volume detecting module, a transmission mechanism and a drive mechanism of the negative pressure wound therapy system with an alarm for exudates flow velocity provided according to the embodiment II of the disclosure.

The reference numbers in the drawings represent:
1-wound, 2-wound filler, 3-wound enclosure, 4-catheter, 5-fluid collection canister, 51-side wall of the fluid collection canister, 52-inner space of the fluid collection canister, 6-negative pressure source, 7-controller, 8-negative pressure wound therapy device, 9-exudates volume detecting module, 91-transmitting module, 92-receiving unit, 10-screw, 11-motor, 12-display interface.

### DETAILED DESCRIPTION

The negative pressure wound therapy system with an alarm for exudates flow velocity provided by the disclosure may be specified in the following in conjunction with embodiments and drawings.

### Embodiment I

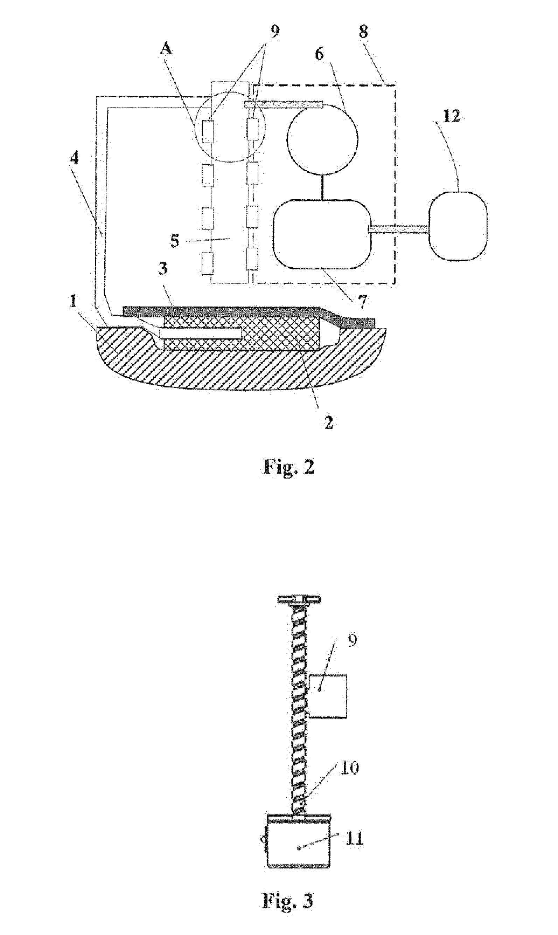

As shown in Figure 2, the negative pressure wound therapy system with an alarm for exudates flow velocity in this embodiment of the disclosure includes:
a fluid collection canister 5, adapted to collect wound exudates;
a negative pressure wound therapy device 8, including a negative pressure source 6 and a controller 7;
a wound kit, adapted to fill and enclose the wound, including a wound filler 2 and a wound enclose 3, where the wound filler 2 is in communication with the fluid collection canister 5 via a catheter 4;
four exudates volume detecting modules 9, set at the side wall of the fluid collection canister 5, where the exudates volume detecting module includes:
a transmitting unit, adapted to transmit electromagnetic wave or ultrasonic wave signal(s) to the fluid collection canister 5, where the transmitting unit is set at one side of the fluid collection canister 5;
a receiving unit, adapted to receive the electromagnetic wave or the ultrasonic wave signal(s) refracted through the fluid collection canister 5, where the receiving unit is set at the other side of the fluid collection canister 5 opposite to the transmitting unit 91 in a way that the receiving unit may receive the electromagnetic wave or the ultrasonic wave signal(s) passed through and refracted by the fluid collection canister 5 if there exist exudates where the receiving unit is located; and
a detecting control unit, adapted to determine whether the receiving unit receives the electromagnetic wave or the ultrasonic wave signal(s), where if the receiving unit does not receive the electromagnetic wave or the ultrasonic wave signal(s), it may be determined that there exist exudates where the detecting module is located.

The four exudates volume detecting modules set vertically along the fluid collection canister determine the change of the volume of the exudates and record elapsed duration.

The controller 7 in this embodiment includes a calculating unit and a processing unit, where the calculating unit may figure out the exudates flow velocity according to the volume of the exudates and the elapsed duration; the processing unit may compare the current exudates flow velocity with a preset value and send out an alarm instruction if the current flow velocity exceeds the preset value.

An alarming apparatus is also provided according to the embodiment, which is adapted to send out a warning signal according to the alarm instruction sent from the processing unit. The alarming apparatus includes a display interface, an LED and a buzzer. The display interface 12 is adapted to display warning information, the LED is adapted to emit warning lights in a corresponding color, and the buzzer is adapted to alarm by emitting a sound at a certain frequency.

The display interface is further adapted to display data of the exudates flow rate selectively. The exudates flow rate may be measured in any one unit from mL/S, mL/Min, mL/H, L/S, L/Min and L/H.

In this embodiment, the liquid volume may be detected on the levels of 100ML, 200ML, 300ML, 400ML, 500ML and 800ML, and the values of these volumes may be recorded. In the calculation of the flow rate, the duration in which the exudate level rises by 100ML may be recorded, hereby the volume of the exudates flowing into the fluid collection canister per unit time may be figured out.

In this embodiment, the alarm includes two levels. The processing unit compares the current flow velocity with a preset value. If the current flow velocity exceeds a first preset value, the processing unit may send out a warning, the LCD display interface may display alarm information, the LED may emit warning lights, and then the medical staff may be reminded to take the appropriate actions. If the current flow velocity exceeds a second warning value, the processing unit may send out an alarm instruction, the LCD display interface may display warning information, the LED may emit warning lights, a buzzer may send out a warning sound, and then the medical staff may be reminded to suspend the treating and take the appropriate emergency actions.

### Embodiment II

This embodiment differs from the embodiment I in that:

There is only one exudates volume detecting module 9, and the transmitting unit and the receiving unit are integrated. As shown in Figure 3, the exudates volume detecting module 9 is sleeved on a screw 10 of which the center line extends vertically. The center line of the screw 10 is parallel to the fluid collection canister 5 vertically. Preferably, the exudates volume detecting module 9 is restrained in the radial direction of the center line of the screw 10, i.e., the exudates volume detecting module 9 may only be moved along the center line of the screw 10. One end of the screw 10 is connected to the motor 11, and the screw 10 may be driven by the motor 11 to rotate around the center line of the screw 10, hence the exudates volume detecting module 9 may be brought to move along the center line of the screw 10 vertically to detect whether there exist exudates at a corresponding position on the fluid collection canister 5.

The screw 10 may be replaced by other transmission mechanisms such as gear set, belt or belt pulley; the liquid volume at the corresponding position on the fluid collection canister 5 may be detected, as long as the exudates volume detecting module 9 can be moved vertically with respect to the fluid collection canister 5.

In this embodiment, driven by the motor 11, the screw 10 may bring the exudates volume detecting module 9 to move vertically. When the receiving unit switches from the state of the infrared signal being detected to the state of the infrared signal being not detected, it may be determined by the exudates volume detecting module 9 that the current position corresponds to the liquid level, and then the liquid level in the fluid collection canister 5 may be obtained according to the stroke of the motor, hence the volume of the exudates in the fluid collection canister 5 may be figured out.

In this embodiment, by the motor 11 and the screw 10, the exudates volume detecting module 9 may detect the different liquid levels in the fluid collection canister. For the calculation of the data regarding flow rate, the change of the liquid level in the fluid collection canister within a set time period may be detected, and the volume of the exudates flowing into the fluid collection canister per unit time may be figured out by the calculating unit, hence the current exudates flow velocity may be obtained. The processing unit compares the current flow velocity with a preset value. If the current flow velocity exceeds a first preset value, the processing unit may sent out a warning, the LCD display interface may display warning information, the LED may emit warning lights, and then the medical staff may be reminded to take the appropriate measures. If the current flow velocity exceeds a second warning value, the processing unit may send out an alarm instruction, the LCD display interface may display alarming information, and the LED may emit warning lights, a buzzer may send out a warning sound, and then the medical staff may be reminded to suspend the treating and take the appropriate emergency measures.

Detailed description is set forth above, and the principle and implementation of the disclosure are described in conjunction with embodiments. The embodiments are only to help better understanding of the method and the concept of the disclosure. It should be noted that modifications and improvements may be made by those skilled in the art within the principle of the disclosure and those modifications and improvements fall into the scope of the claims of the disclosure.

## Claims

1. A negative pressure wound therapy system with an alarm for exudates flow velocity, comprising:
a fluid collection canister adapted to collect wound exudates; a negative pressure wound therapy device comprising a negative pressure source and a controller; a wound kit adapted to fill and enclose the wound, wherein the wound kit is in communication with the fluid collection canister via a catheter, wherein the system further comprises a flow velocity alarm processing module and an alarming apparatus, the flow velocity alarm processing module is adapted to send out an alarm instruction in a case that the exudates flow velocity exceeds a preset value, and the alarming apparatus is adapted to send out a warning signal according to the alarm instruction.

2. The negative pressure wound therapy system with an alarm for exudates flow velocity according to claim 1, wherein the flow velocity alarm processing module comprises: a monitoring unit adapted to monitor a volume of the exudates in the fluid collection canister and to monitor an elapsed time duration; an calculating unit adapted to figure out a current exudates flow velocity according to a change of the volume of the exudates within a set time period; and a processing unit adapted to send out an alarm instruction in a case that the current flow velocity exceeds a preset value.

3. The negative pressure wound therapy system with an alarm for exudates flow velocity according to claim 1, wherein the preset value comprises a first preset value and a second preset value, wherein the flow velocity alarm processing module is adapted to send out a first alarm instruction if the exudates flow velocity exceeds the first preset value; the alarming apparatus is adapted to send out the warning signal according to the first alarm instruction; the flow velocity alarm processing module is further adapted to send out a second alarm instruction if the exudates flow velocity exceeds the second preset value; and the alarming apparatus is further adapted to send out the warning signal according to the second alarm instruction.

4. The negative pressure wound therapy system with an alarm for exudates flow velocity according to claim 1, wherein the alarming apparatus comprises an LED adapted to emit lights in a corresponding color after receiving the alarm instruction.

5. The negative pressure wound therapy system with an alarm for exudates flow velocity according to claim 1, wherein the alarming apparatus comprises a buzzer adapted to send out a sound at a preset frequency after receiving the alarm instruction.

6. The negative pressure wound therapy system with an alarm for exudates flow velocity according to claim 1, wherein the alarming apparatus comprises an LCD display interface adapted to display alarming information after receiving the alarm instruction.

7. The negative pressure wound therapy system with an alarm for exudates flow velocity according to claim 1, wherein the alarming apparatus comprises:
an LED, adapted to emit lights in a corresponding color after receiving the alarm instruction; and
a buzzer, adapted to send out a sound at a preset frequency after receiving the alarm instruction.

8. The system according to claim 1, wherein the alarming apparatus comprises:
an LED, adapted to emit lights in a corresponding color after receiving the alarm instruction; and
an LCD display interface, adapted to display alarming information after receiving the alarm instruction.

9. The system according to claim 1, wherein the alarming apparatus comprises:
a buzzer, adapted to send out a sound at a preset frequency after receiving the alarm instruction; and
an LCD display interface, adapted to display alarming information after receiving the alarm instruction.

10. The negative pressure wound therapy system with an alarm for exudates flow velocity according to claim 1, wherein the alarming apparatus comprises:
an LED, adapted to emit lights in a corresponding color after receiving the alarm instruction;
an LCD display interface, adapted to display alarming information after receiving the alarm instruction; and
a buzzer, adapted to send out a sound at a preset frequency after receiving the alarm instruction.
